(19) Europäisches Patentamt
European Patent Office
Office européen des brevets

(11) Veröffentlichungsnummer : **0 061 051**
**B1**

(12) # EUROPÄISCHE PATENTSCHRIFT

(45) Veröffentlichungstag der Patentschrift : **18.09.85**

(51) Int. Cl.⁴ : **C 07 D405/06**, C 07 D249/08

(21) Anmeldenummer : **82101800.9**

(22) Anmeldetag : **08.03.82**

(54) **Substituierte Triazolylmethyl-oxirane, Verfahren zu ihrer Herstellung sowie ihre Verwendung als Zwischenprodukte.**

(30) Priorität : **21.03.81 DE 3111238**

(43) Veröffentlichungstag der Anmeldung : **29.09.82 Patentblatt 82/39**

(45) Bekanntmachung des Hinweises auf die Patenterteilung : **18.09.85 Patentblatt 85/38**

(84) Benannte Vertragsstaaten : **AT BE CH DE FR GB IT LI NL**

(56) Entgegenhaltungen :
**EP-A- 0 026 312**
**EP-A- 0 044 422**

(73) Patentinhaber : **BAYER AG**
**Konzernverwaltung RP Patentabteilung**
**D-5090 Leverkusen 1 Bayerwerk (DE)**

(72) Erfinder : **Holmwood, Graham, Dr.**
**Katernberger Strasse 184**
**D-5600 Wuppertal 1 (DE)**
Erfinder : **Regel, Erik**
**Bergerheide 72a**
**D-5600 Wuppertal 1 (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 061 051 B1

**Beschreibung**

Die vorliegende Erfindung betrifft neue substituierte Triazolylmethyl-oxirane, mehrere Verfahren zur deren Herstellung sowie die Verwendung der neuen substituierten Triazolylmethyl-oxirane als Zwischenprodukte zur Herstellung von Pflanzenschutzmitteln.

Es ist bereits bekannt geworden, daß 2-Chlorethyltrimethyl-ammoniumchlorid und 2-Chlorethylphosphonsäure pflanzenwuchsregulierende Eigenschaften aufweisen, (vgl. US-A 3 156 554 und DE-A 1 667 968). Allerdings ist die Wirksamkeit dieser Stoffe, vor allem bei niedrigen Aufwandmengen, nicht immer ausreichend.

Weiterhin werden in der am 18. März 1981 eingereichten Britischen Patentanmeldung 08 412/1981, die eine der drei prioritätsbegründenden Anmeldungen der EP-A 0 061 835 darstellt, durch die dort angegebene allgemeine Formel (VI) Azolyl-alkyloxirane beschrieben.

In der betreffenden Formel kann eines der beiden Kohlenstoffatome des Oxiranringes sowohl eine Triazolyl-Gruppe als auch einen gegebenenfalls substituierten Alkylrest tragen. Es werden jedoch keine speziellen Verbindungen dieses Typs offenbart.

Es wurden nun als neue Verbindungen die substituierten Triazolylmethyl-oxirane der Formel

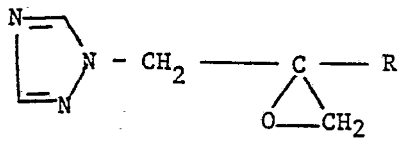

(I)

in welcher R für gegebenenfalls substituiertes Alkyl der Formel

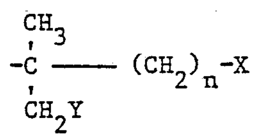

steht, wobei

X für Wasserstoff, Halogen, Alkoxy mit 1 bis 4 Kohlenstoffatomen, Alkythio mit 1 bis 4 Kohlenstoffatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cyano, Alkoxycarbonyl mit 1 bis 4 Kohlenstoffatomen im Alkoxyteil, für gegebenenfalls durch Fluor, Chlor, Methyl und/oder Ethyl substituiertes Phenyl, für gegebenenfalls durch Fluor, Chlor, Methyl und/oder Ethyl substituiertes Phenoxy oder für gegebenenfalls durch Fluor, Chlor, Methyl und/oder Ethyl substituiertes Benzyloxy steht,

Y für Wasserstoff oder Halogen steht und

n für 0 oder 1 steht,

gefunden.

Weiterhin wurde gefunden, daß man die substituierten Triazolylmethyl-oxirane der Formel (I) erhält, wenn man Triazolylketone der Formel

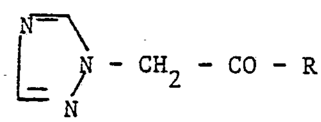

(II)

in welcher R die oben angegebene Bedeutung hat,

a) mit Dimethyloxosulfonium-methylid der Formel

$$\delta\oplus\ \delta\ominus$$

$$(CH_3)_2SOCH_2 \qquad \text{(III)}$$

in Gegenwart eines Verdünnungsmittels, oder

b) mit Trimethylsulfonium-methylsulfat der Formel

$$[(CH_3)_3S]CH_3SO_4 \qquad \text{(IV)}$$

in Gegenwart eines inerten organischen Lösungsmittels sowie in Gegenwart einer Base umsetzt.

Die erfindungsgemäßen substituierten Triazolylmethyloxirane der Formel (I) eignen sich als Zwischenprodukte zur Synthese von 1-Hydroxyethyl-triazol-Derivaten, welche pflanzenwuchsregulierende und fungizide Eigenschaften besitzen.

Überraschenderweise sind die 1-Hydroxyethyl-triazol-Derivate, die sich aus den erfindungsgemäßen substituierten Triazolylmethyl-oxiranen der Formel (I) durch Umsetzung mit Phenolen herstellen lassen, den vorbekannten, gut wirksamen Verbindungen 2-Chlorethyltrimethyl-ammoniumchlorid und 2-Chlorethylphosphonsäure bezüglich ihrer pflanzenwuchsregulierenden Eigenschaften überlegen. Die erfindungsgemäßen Stoffe stellen somit als Zwischenprodukte zur Synthese hochwertiger Pflanzenwachstumsregulatoren und Fungizide eine wesentliche Bereicherung der Technik dar.

Die erfindungsgemäßen Verbindungen sind dadurch charakterisiert, daß sie einen Oxiran-Ring enthalten, der an einem der beiden Ring-Kohlenstoffatome geminal substituiert ist durch eine (1,2,4-Triazol-1-yl)-methyl-Gruppe und durch einen verzweigten gegebenenfalls substituierten Alkylrest. Diese Stoffe werden zwar von der allgemeinen Formel (VI) umfaßt, die in der vorgängigen, aber nicht vorveröffentlichten Britischen Patentanmeldung 08 412/1981 (vgl. EP-A 0 061 835) aufgeführt ist. Es werden aber keine Verbindungen speziell erwähnt, in denen der dort als $R^1$ bezeichnete Rest für eine verzweigte, gegebenenfalls substituierte Alkylgruppe steht. Damit unterscheiden sich die erfindungsgemäßen substituierten Oxirane der Formel (I) eindeutig von den konstitutionell ähnlichsten vorbeschriebenen Substanzen.

Im einzelnen seien außer den bei den Herstellungsbeispielen genannten Verbindungen folgende Verbindungen der allgemeinen Formel (I) genannt :

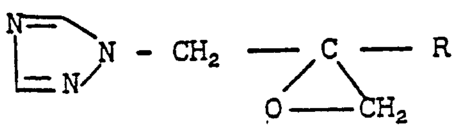

(I)

| R | R |
|---|---|
| $-C(CH_3)_2-CH_2Cl$ | $-C(CH_3)_2-CH_2F$ |
| $-CH(CH_2Cl)_2-CH_3$ | $-C(CH_2F)_2-CH_3$ |
| $-C(CH_3)_2-COOCH_3$ | $-C(CH_3)_2-COOC_2H_5$ |
| $-C(CH_3)_2-$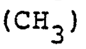$-Cl$ | $-C(CH_3)_2-O-$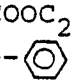$-Cl$ |
| $-C(CH_3)_2-CH_2-$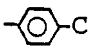$-Cl$ | $-C(CH_3)_2-CH_2-O-$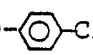$-Cl$ |
| $-(CH_3)_2-CH_2-O-CH_3$ | $-C(CH_3)_2-CH_2-O-C_2H_5$ |
| $-C(CH_3)_2-SCF_3$ | $-C(CH_3)_2-CH_2-CN$ |
| $-CH(CH_3)_2$ | |

Verwendet man beispielsweise 3,3-Dimethyl-1-(1,2,4-triazol-1-yl)-butan-2-on sowie Trimethyloxosulfoniumiodid und Natriumhydrid (in-situ-Herstellung von Dimethyloxosulfonium-methylid) als Ausgangsstoffe, so kann der Reaktionsablauf durch das folgende Formelschema wiedergegeben werden (Verfahrensvariante a) :

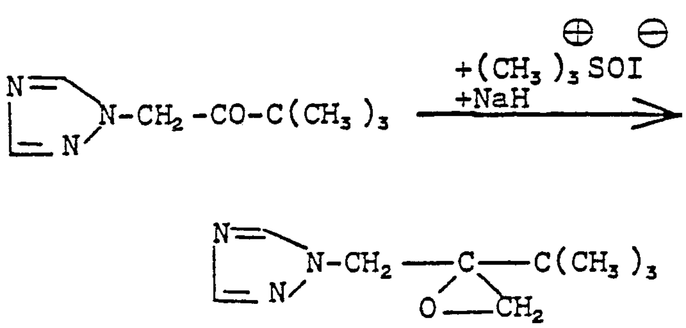


Die für die erfindungsgemäßen Verfahren (a) und (b) als Ausgangsstoffe zu verwendenden Triazolylketone sind durch die Formel (II) allgemein definiert. In dieser Formel steht R für diejenigen Reste, die bei der Beschreibung der erfindungsgemäßen Stoffe der Formel (I) bereits für diesen Substituenten genannt wurden.

Verwendet man beispielsweise 3,3-Dimethyl-1-(1,2,4-triazol-1-yl)-butan-2-on sowie Dimethylsulfid und Dimethylsulfat (in-situ-Herstellung von Trimethylsulfonium-methyl-sulfat) als Ausgangsstoffe und Natriummethylat als Base, so kann der Reaktionsablauf durch das folgende Formelschema wiedergegeben werden (Verfahrensvariante b) :

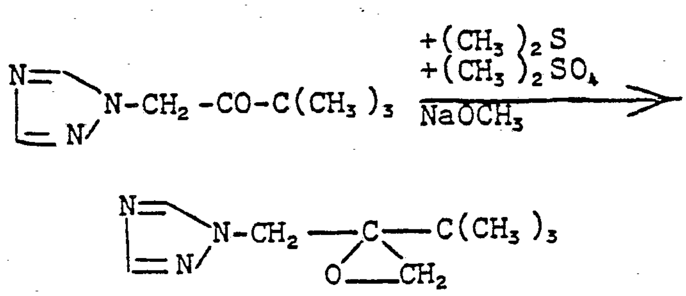


Die Triazolylketone der Formel (II) sind teilweise bekannt (vergleiche z. B. DE-A 2 431 407, DE-A 2 610 022, DE-A 2 638 470, DE-A 2 820 361), teilweise sind sie Gegenstand einer eigenen älteren Anmeldung. Sie werden erhalten, indem man Halogenketone der Formel

$$Hal—CH_2—CO—R \qquad (V)$$

in welcher

R die oben angegebene Bedeutung hat und

Hal für Chlor oder Brom steht,

mit 1,2,4-Triazol in Gegenwart eines Verdünnungsmittels, wie beispielsweise Aceton, und in Gegenwart eines Säurebindemittels, wie beispielsweise Kaliumcarbonat, bei Temperaturen zwischen 20 und 150 °C umsetzt.

Die Halogenketone der Formel (V) werden erhalten, indem man Ketone der Formel

$$CH_3—CO—R \qquad (VI)$$

in welcher R die oben angegebene Bedeutung hat, in einem inerten organischen Lösungsmittel bei Raumtemperatur mit Chlor oder Brom versetzt ; oder z. B. mit üblichen Chlorierungsmitteln, wie Sulfurylchlorid, bei 20 bis 60 °C umsetzt.

Das außerdem für die Verfahrensvariante (a) als Ausgangsstoff benötigte Dimethylsulfoniummethylid der Formel (III) ist bekannt (vergleiche J. Amer. Chem. Soc. 87, 1363-1364)). Es wird in frisch zubereitetem Zustand verarbeitet, indem man es in situ durch Umsetzung von Trimethyloxosulfoniumiodid mit Natriumhydrid bzw. Natriumamid in Gegenwart eines Verdünnungsmittels erzeugt.

Das außerdem für die Verfahrensvariante (b) als Ausgangsstoff benötigte Trimethylsulfonium-methylsulfat der Formel (IV) ist ebenfalls bekannt (vergleiche Heterocycles 8, 397 (1977)). Es wird ebenfalls in frisch hergestelltem Zustand eingesetzt, indem man es durch Reaktion von Dimethylsulfid mit Dimethylsulfat in-situ erzeugt.

Als Verdünnungsmittel kommen für die erfindungsgemäße Verfahrensvariante (a) vorzugsweise Dimethylsulfoxid sowie Gemische von Dimethylsulfoxid mit anderen inerten organischen Lösungsmitteln, wie z. B. Tetrahydrofuran, in Frage.

Die Reaktionstemperaturen können bei der Durchführung der Verfahrensvariante (a) innerhalb eines größeren Bereiches variiert werden. Im allgemeinen arbeitet man bei Temperaturen zwischen − 10 °C und 80 °C, vorzugsweise zwischen 0 und 50 °C.

Die Durchführung des erfindungsgemäßen Verfahrens (a) und die Aufarbeitung des anfallenden Reaktionsgemisches erfolgen nach üblichen Methoden (vergleiche J. Amer. Chem. Soc. 87, 1363-1364 (1965)).

Als inertes organisches Lösungsmittel kommt für die erfindungsgemäße Verfahrensvariante (b) vorzugsweise Acetonitril in Frage.

Als Base können bei der erfindungsgemäßen Verfahrensvariante (b) übliche starke anorganische oder organische Basen verwendet werden, wie vorzugsweise Natriummethylat oder Kalium-tert.-butylat.

Die Reaktionstemperaturen können bei der Durchführung der Verfahrensvariante (b) innerhalb eines größeren Bereiches variiert werden. Im allgemeinen arbeitet man bei Temperaturen zwischen 0 °C und 60 °C, vorzugsweise bei Raumtemperatur.

Die Durchführung der erfindungsgemäßen Verfahrensvariante (b) und die Aufarbeitung des anfallenden Reaktionsgemisches erfolgen nach üblichen Methoden (vergleiche Heterocycles 8, 397 (1977)).

Wie schon oben erwähnt, eignen sich die erfindungsgemäßen substituierten Triazolylmethyl-oxirane der Formel (I) als Zwischenprodukte zur Synthese von 1-Hydroxyethyltriazol-Derivaten, welche starke pflanzenwuchsregulierende und fungizide Eigenschaften besitzen.

So lassen sich zum Beispiel 1-Hydroxyethyl-triazol-Derivate der Formel

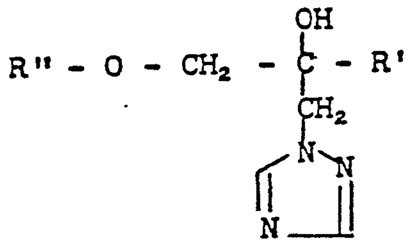

(VII)

in welcher
R' für Alkyl steht und
R'' für gegebenenfalls substituiertes Phenyl steht,
herstellen, indem man substituierte Triazolylmethyloxirane der Formel

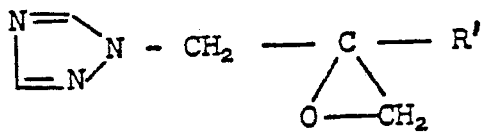

(Ia)

in welcher R' die oben angegebene Bedeutung hat,
mit Phenolen der Formel

R''—OH (VIII)

in welcher R'' die oben angegebene Bedeutung hat,
in Gegenwart eines inerten organischen Lösungsmittels, wie beispielsweise Ethanol, und gegebenenfalls in Gegenwart einer Base, wie beispielsweise Natriummethylat, gegebenenfalls unter einem Druck von 1 bis 25 bar, bei Temperaturen zwischen 60 °C und 150 °C umsetzt. Die Durchführung dieser Reaktion und die Aufarbeitung des anfallenden Reaktionsgemisches erfolgen nach üblichen Methoden (vgl. Herstellungsbeispiele).

In den Verbindungen der Formel (Ia) steht R' vorzugsweise für verzweigtes Alkyl mit bis zu 4 Kohlenstoffatomen.

Die bei der obigen Umsetzung als Reaktionskomponenten benötigten Phenole sind durch die Formel (VIII) allgemein definiert. In dieser Formel steht R'' vorzugsweise für Phenyl, welches ein-, zwei- oder dreifach, gleichartig oder verschieden substituiert sein kann durch Halogen, Alkyl mit 1 bis 4 Kohlenstoffatomen, Cycloalkyl mit 5 bis 7 Kohlenstoffatomen, Alkoxy und Alkylthio mit jeweils 1 bis 4 Kohlenstoffatomen, Halogenalkyl und Halogenalkoxy sowie Halogenalkylthio mit jeweils 1 bis 2 Kohlenstoff- und 1 bis 5 gleichen oder verschiedenen Halogenatomen, wie insbesondere Fluor- und Chloratomen sowie für jeweils gegebenenfalls substituiertes Phenyl, Phenoxy und Phenylalkyl sowie Phenylalkoxy mit 1 bis 2 Kohlenstoffatomen im Alkylteil bzw. im Alkoxyteil, wobei als Substituenten vorzugsweise genannt seien : Halogen und Alkyl mit 1 bis 4 Kohlenstoffatomen.

Die Phenole der Formel (VIII) sind allgemein bekannte Verbindungen der organischen Chemie.

## Herstellungsbeispiele

### Beispiel 1

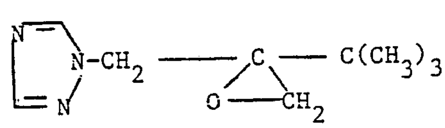

(Verfahrensvariante a)

Unter trockener Stickstoff-Atmosphäre werden 6,0 g Natriumhydrid (80 %ig in Paraffinöl) und 44 g Trimethylsulfoxoniumiodid vorgelegt und bei 10 bis 15 °C Innentemperatur mit 200 ml absolutem Dimethylsulfoxid langsam versetzt.

Nachdem die Wasserstoff-Etnwicklung abgeklungen ist, wird 15 Minuten nachgerührt, und dann wird eine Lösung von 30,1 g 3,3-Dimethyl-1-(1,2,4-triazol-1-yl)-butan-2-on in 50 ml absolutem Tetrahydrofuran unter Kühlung auf 20 °C Innentemperatur eingetropft. Anschließend wird 1 1/2 Stunden bei 50 °C nachgerührt.

Die Lösungsmittel werden im Hochvakuum abgezogen, der Rückstand mit gesättigter Kochsalzlösung versetzt und sechsmal mit Essigester extrahiert. Die vereinigten Essigesterphasen werden über Natriumsulfat getrocknet, eingeengt und der Rückstand wird im Hochvakuum destilliert. Man erhält 23,5 g (72 % der Theorie) 2-tert.-Butyl-2-(1,2,4-triazol-1-yl)-methyl-oxiran vom Siedepunkt 68 bis 72 °C/0,008 mbar und einem Berechnungsindex von $n_D^{20}$ = 1,480 9

Beispiel 2

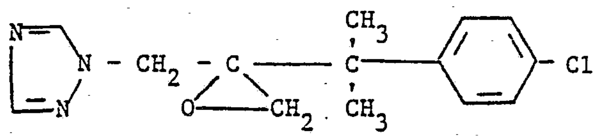

(Verfahrensvariante a)

Unter Stickstoff-Atmosphäre werden 3,6 g (0,12 Mol) Natriumhydrid (80 %ig in Paraffinöl) und 26,5 g (0,12 Mol) Trimethylsulfoxoniumiodid bei 10 °C gemischt und langsam mit 100 ml Dimethylsulfoxid versetzt. Nach einer Stunde werden in die Suspension 26,35 g (0,1 Mol) 3-(4-Chlorphenyl)-3-methyl-1-(1,2,4-triazol-1-yl)-butan-2-on, gelöst in 50 ml Dimethylsulfoxid, zugetropft.

Das Gemisch wird 2 Tage bei Raumtemperatur gerührt, dann 5 Stunden auf 60 °C erhitzt und in 3 000 ml Wasser eingerührt. Das ausgeschiedene Öl wird in Chloroform aufgenommen, mit Wasser gewaschen, über Natriumsulfat getrocknet und im Vakuum eingedampft.

Man erhält 21 g (76 % der Theorie) 2-(4-Chlor-α,α-dimethylbenzyl)-2-(1,2,4-triazol-1-yl)-methyl-oxiran vom Brechungsindex $n_D^{20}$ = 1,541 2.

Die in der folgenden Tabelle 2 aufgeführten Stoffe werden nach den in den Beispielen 1 und 2 beschriebenen Methoden hergestellt:

Tabelle 2

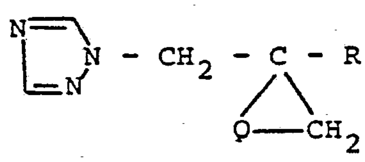

(I)

| Beispiel Nr. | R | Physikalische Kontante |
|---|---|---|
| 3 | $-C(CH_3)_2CH_2F$ | $n_D^{20}$ = 1,4805 |
| 4 | $-C(CH_3)_2CH_2OCH_3$ | Kp.: 90-92°C/ 0,008 mbar |
| 5 | $-C(CH_3)_2CH_2OC_2H_5$ | Kp.: 83-85°C/ 0,007 mbar |
| 6 | 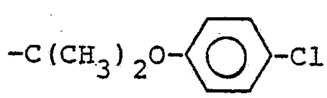 | Fp.: 70-71°C |

Herstellung von Endprodukten

Beispiel VII-1

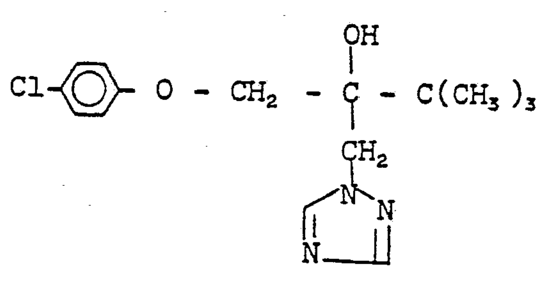

9,9 g 4-Chlorphenol werden zu einer Lösung von 0,39 g Natrium in 130 ml absolutem Ethanol gegeben. Anschließend werden 10,8 g 2-tert.-Butyl-2-(1,2,4-triazol-1-yl)-methyl-oxiran, gelöst in 34 ml absolutem Ethanol, zugegeben, und das Reaktionsgemisch wird über Nacht unter Rückfluß erhitzt.

Danach wird das Reaktionsgemisch eingeengt, der Rückstand in Essigester aufgenommen und einmal mit Wasser, einmal mit 1 n Natronlauge, danach zweimal mit Wasser und einmal mit gesättigter Kochsalzlösung gewaschen.

Die Essigesterphase wird über Natriumsulfat getrocknet und eingeengt. Der Rückstand wird über eine Kieselgelsäule chromatographiert (Laufmittel : Dichlormethan/Essigester = 2 : 1). Nach Umkristallisation aus Ligroin erhält man 13,0 g (70,5 % der Theorie) 2-(âChlor-phenoxy) methyl-3,3-dimethyl-1-(1,2,4-triazol-1-yl) butan-2-ol vom Schmelzpunkt 89-91 °C.

In analoger Weise werden die in der folgenden Tabelle formelmäßig aufgeführten Stoffe der Formel (VII) erhalten.

Tabelle 1

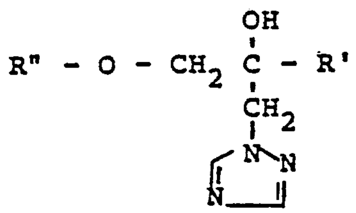

(VII)

| Beispiel Nr. | R" | R' | Schmelzpunkt (°C) |
|---|---|---|---|
| VII-2 | 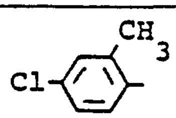 | $-C(CH_3)_3$ | 125,5 - 129 |
| VII-3 | 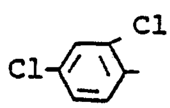 | $-C(CH_3)_3$ | 120,5 - 123,5 |
| VII-4 | 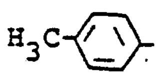 | $-C(CH_3)_3$ | 98 - 101,5 |
| VII-5 | 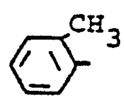 | $-C(CH_3)_3$ | 89 - 101 |

Die gute pflanzenwuchsregulierende Wirksamkeit von 1-Hydroxyethyl-triazol-Derivaten der Formel (VII) geht aus den folgenden Beispielen hervor.

In diesen Beispielen werden die nachstehend angegebenen Verbindungen als Vergleichssubstanzen eingesetzt :

(A) = 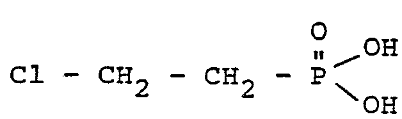

(2-Chlorethyl-phosphonsäure)

(B) = $Cl - CH_2 - CH_2 - \overset{\oplus}{N}(CH_3)_3 \quad Cl^{\ominus}$

(2-Chlorethyl-trimethyl-ammoniumchlorid).

Beispiel A

Wuchshemmung bei Zuckerrüben

Lösungsmittel : 30 Gewichtsteile Dimethylformamid
Emulgator : 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Zuckerrüben werden im Gewächshaus bis zur vollen ausbildung der Keimblätter angezogen. In diesem Stadium werden die Pflanzen tropfnaß mit den Wirkstoffzubereitungen besprüht. Nach 14 Tagen wird der Zuwachs der Pflanzen gemessen und die Wuchshemmung in Prozent des Zuwachses der Kontrollpflanzen berechnet. Es bedeuten 0 % Wuchshemmung ein Wachstum entsprechend dem der Kontrollpflanzen. 100 % Wuchshemmung bedeutet Stillstand des Wachstums.

Die Wirkstoffe VII-1 und VII-4 zeigen in diesem Test bessere Wuchshemmung als die aus dem Stand der Technik bekannte Substanz (B).

Beispiel B

Wuchshemmung bei Sojabohnen

Lösungsmittel : 10 Gewichtsteile Methanol
Emulgator : 2 Gewichtsteile Polyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Junge Sojabohnen werden in dem Stadium, in dem die ersten Folgeblätter entfaltet sind, mit den Wirkstoffzubereitungen tropfnaß besprüht. Nach 2 Wochen wird der Zuwachs gemessen und die Wuchshemmung in % des Zuwachses der Kontrollpflanzen berechnet. Es bedeuten 100 % den Stillstand des Wachstums und 0 % ein Wachstum entsprechend dem der unbehandelten Kontrollpflanzen.

Die Wirkstoffe VII-1 und VII-2 zeigen in diesem Test eine bessere Wuchshemmung als die aus dem Stand der Technik bekannte Substanz (B).

Beispiel C

Wuchshemmung bei Baumwolle

Lösungsmittel : 30 Gewichsteile Dimethylformamid
Emulgator : 1 Gewichsteil Polyethylen-Sorbitan-Monolaurat

Zur Herstellung einer zweckmäßigen Wirkstoffzubereitung vermischt man 1 Gewichtsteil Wirkstoff mit der angegebenen Menge Lösungsmittel und Emulgator und füllt mit Wasser auf die gewünschte Konzentration auf.

Baumwollpflanzen werden im Gewächshaus bis zur vollen Entfaltung des 5. Folgeblattes angezogen. In diesem Stadium werden die Pflanzen tropfnaß mit den Wirkstoffzubereitungen besprüht. Nach 3 Wochen wird der zuwachs der Pflanzen gemessen und die Wuchshemmung in Prozent des Zuwachses der Kontrollpflanzen berechnet. Es bedeuten 100 % Wuchshemmung den Stillstand des Wachstums und 0 % ein Wachstum entsprechend dem der Kontrollpflanzen.

Die Wirkstoffe VII-1 und VII-4 zeigen in diesem Test eine bessere Wuchshemmung als die aus dem Stand der Technik bekannte Substanz (A).

**Patentansprüche**

1. Substituierte Triazolylmethyl-oxirane der Formel

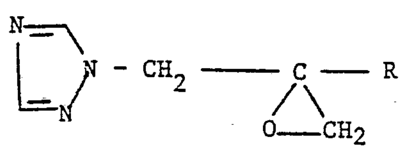

(I)

in welcher R für gegebenenfalls substituiertes Alkyl der Formel

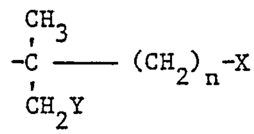

steht, wobei

X für Wasserstoff, Halogen, Alkoxy mit 1 bis 4 Kohlenstoffatomen, Alkylthio mit 1 bis 4 Kohlenstoffatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cyano, Alkoxycarbonyl mit 1 bis 4 Kohlenstoffatomen im Alkoxyteil, für gegebenenfalls durch Fluor, Chlor, Methyl und/oder Ethyl substituiertes Phenyl, für gegebenenfalls durch Fluor, Chlor, Methyl und/oder Ethyl substituiertes Phenoxy oder für gegebenenfalls durch Fluor, Chlor, Methyl und/oder Ethyl substituiertes Benzyloxy steht,

Y für Wasserstoff oder Halogen steht und

n für 0 oder 1 steht.

2. Substituiertes Triazolylmethyl-oxiran der Formel

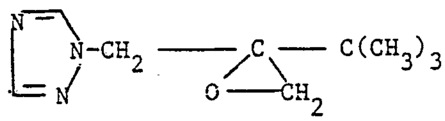

3. Substituiertes Triazolylmethyl-oxiran der Formel

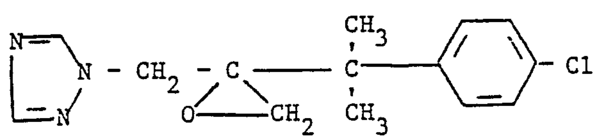

4. Verfahren zur Herstellung von substituierten Triazolylmethyl-oxiranen der Formel

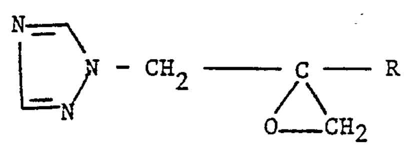

(I)

in welcher R für gegebenenfalls substituiertes Alkyl der Formel

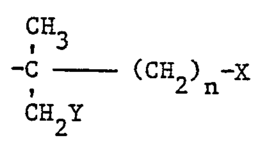

steht, wobei

X für Wasserstoff, Halogen, Alkoxy mit 1 bis 4 Kohlenstoffatomen, Alkylthio mit 1 bis 4 Kohlenstoffatomen, Halogenalkoxy mit 1 oder 2 Kohlenstoffatomen und 1 bis 5 gleichen oder verschiedenen Halogenatomen, Halogenalkylthio mit 1 oder 2 Kohlenstoffatomen und 1 bis 5 gleichen oder verschiedenen Halogenatomen, Cyano, Alkoxycarbonyl mit 1 bis 4 Kohlenstoffatomen im Alkoxyteil, für gegebenenfalls durch Fluor, Chlor, Methyl und/oder Ethyl substituiertes Phenyl, für gegebenenfalls durch Fluor, Chlor, Methyl und/oder Ethyl substituiertes Phenoxy oder für gegebenenfalls durch Fluor, Chlor, Methyl und/oder Ethyl substituiertes Benzyloxy steht,

Y für Wasserstoff oder Halogen steht und

n für 0 oder 1 steht,

dadurch gekennzeichnet, daß man Triazolylketone der Formel

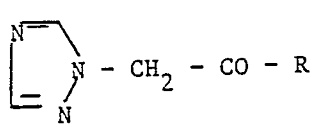

(II)

in welcher R die oben angegebene Bedeutung hat

a) mit Dimethyloxosulfonium-methylid der Formel

$$(CH_3)_2\overset{\delta\oplus\,\delta\ominus}{SOCH_2} \qquad (III)$$

in Gegenwart eines Verdünnungsmittels, oder

b) mit Trimethylsulfonium-methylsulfat der Formel

$$[(CH_3)_3S]^{\oplus}\, CH_3SO_4^{\ominus} \qquad (IV)$$

in Gegenwart eines organischen Lösungsmittels sowie in Gegenwart einer Base umsetzt.

5. Verwendung von substituierten Triazolylmethyloxiranen der Formel (I) als Zwischenprodukte zur Synthese von Stoffen mit pflanzenwuchsregulierender und fungizider Wirksamkeit.

**Claims**

1. Substituted triazolylmethyl-oxiranes of the formula

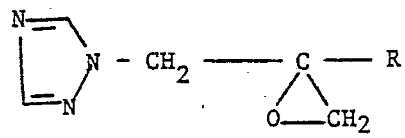

(I)

in which R represents optionally substituted alkyl of the formula

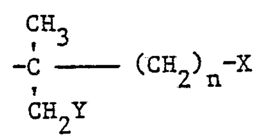

wherein

X represents hydrogen, halogen, alkoxy with 1 to 4 carbon atoms, alkylthio with 1 to 4 carbon atoms, halogenoalkoxy with 1 or 2 carbon atoms and 1 to 5 identical or different halogen atoms, halogenoalkylthio with 1 or 2 carbon atoms and 1 to 5 identical or different halogen atoms, cyano, alkoxycarbonyl with 1 to 4 carbon atoms in the alkoxy part, phenyl optionally substituted by fluorine, chlorine, methyl and/or ethyl, phenoxy optionally substituted by fluorine, chlorine, methyl and/or ethyl or benzyloxy optionally substituted by fluorine, chlorine, methyl and/or ethyl,

Y represents hydrogen or halogen and

n represents 0 or 1.

2. The substituted triazolylmethyl-oxirane of the formula

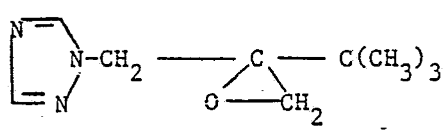

3. The substituted triazolylmethyl-oxirane of the formula

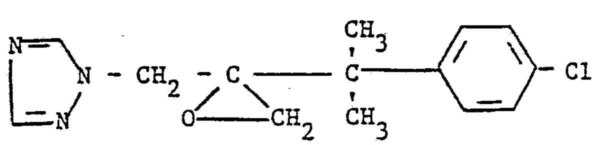

4. Process for the preparation of substituted triazolylmethyloxiranes of the formula

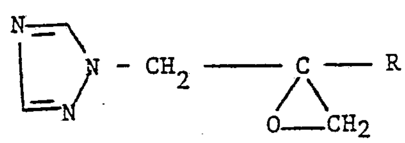

(I)

in which R represents optionally substituted alkyl of the formula

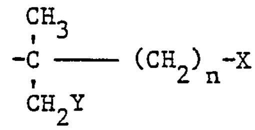

wherein

X represents hydrogen, halogen, alkoxy with 1 to 4 carbon atoms, alkylthio with 1 to 4 carbon atoms, halogenoalkoxy with 1 or 2 carbon atoms and 1 to 5 identical or different halogen atoms, halogenoalkylthio with 1 or 2 carbon atoms and 1 to 5 identical or different halogen atoms, cyano, alkoxycarbonyl with 1 to 4 carbon atoms in the alkoxy part, phenyl optionally substituted by fluorine, chlorine, methyl and/or ethyl, phenoxy optionally substituted by fluorine, chlorine, methyl and/or ethyl or benzyloxy optionally substituted by fluorine, chlorine, methyl and/or ethyl,

Y represents hydrogen or halogen and

n represents 0 or 1,

characterised in that triazolyl-ketones of the formula

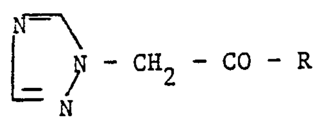

(II)

in which R has the abovementioned meaning are reacted

a) with dimethyloxosulphonium methylide of the formula

$$\overset{\delta\oplus\delta\ominus}{(CH_3)_2SOCH_2} \qquad (III)$$

in the presence of a diluent, or

b) with trimethylsulphonium methylsulphate of the formula

$$[(CH_3)_3S]^{\oplus}\ CH_3SO_4^{\ominus} \qquad (IV)$$

in the presence of an organic solvent and in the presence of a base.

5. Use of substituted triazolylmethyl-oxiranes of the formula (I) as intermediates for the synthesis of compounds having plant-growth-regulating and fungicidal activity.

**Revendications**

1. Triazolylméthyl-oxirannes substitués de formule

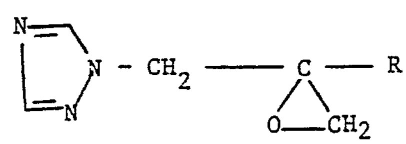

(I)

dans laquelle R représente un groupe alkyle éventuellement substitué de formule

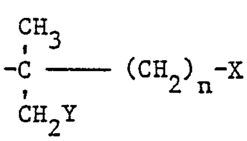

(I)

dans laquelle

X représente l'hydrogène, un halogène, un groupe alcoxy en $C_1$-$C_4$, alkylthio en $C_1$-$C_4$, halogénoalcoxy contenant 1 ou 2 atomes de carbone et 1 à 5 atomes d'halogènes identiques ou différents, halogénoalkylthio contenant 1 ou 2 atomes de carbone et 1 à 5 atomes d'halogènes identiques ou différents, cyano, alcoxy-carbonyle contenant 1 à 4 atomes de carbone dans la partie alcoxy, phényle éventuellement substitué par le fluor, le chlore, des groupes méthyle et/ou éthyle, phénoxy éventuellement substitué par le fluor, le chlore, des groupes méthyle et/ou éthyle, ou benzyloxy éventuellement substitué par le fluor, le chlore, des groupes méthyle et/ou éthyle,

Y représente l'hydrogène ou un halogène et

n est égal à 0 ou 1.

2. Triazolylméthyl-oxiranne substitué de formule

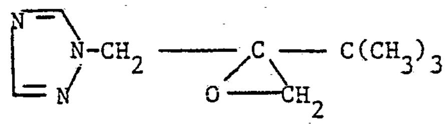

3. Triazolylméthyl-oxiranne substitué de formule

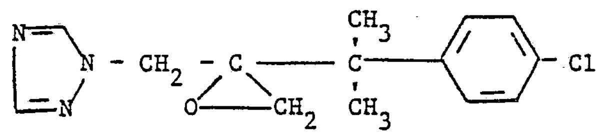

4. Procédé de préparation de triazolylméthyl-oxirannes substitués de formule

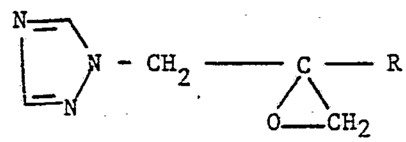

(I)

dans laquelle R représente un groupe alkyle éventuellement substitué de formule

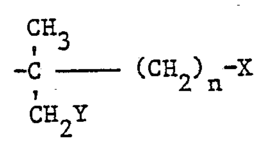

dans laquelle

X représente l'hydrogène, un halogène, un groupe alcoxy en $C_1$-$C_4$, alkylthio en $C_1$-$C_4$, halogénoalcoxy contenant 1 ou 2 atomes de carbone et 1 à 5 atomes d'halogènes identiques ou différents, halogénoalkylthio contenant 1 ou 2 atomes de carbone et 1 à 5 atomes d'halogènes identiques ou différents, cyano, alcoxycarbonyle contenant 1 à 4 atomes de carbone dans la partie alcoxy, phényle éventuellement substitué par le fluor, le chlore, des groupes méthyle et/ou éthyle, phénoxy éventuellement substitué par le fluor, le chlore, des groupes méthyle et/ou éthyle, ou benzyloxy éventuellement substitué par le fluor, le chlore, des groupes méthyle et/ou éthyle,

Y représente l'hydrogène ou un halogène et

n est égal à 0 ou 1,

caractérisé en ce que l'on fait réagir des triazolylcétones de formule

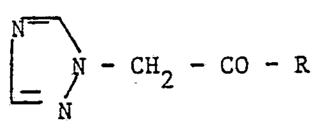

(II)

dans laquelle R a la signification indiquée ci-dessus,

a) avec du méthylure de diméthyloxosulfonium de formule

$$(CH_3)_2\overset{\oplus}{S}O\overset{\ominus}{C}H_2 \qquad (III)$$

en présence d'un diluant, ou bien

b) avec du méthylsulfate de triméthylsulfonium de formule

$$[(CH_3)_3S]^{\oplus}\ CH_3SO_4^{\ominus} \qquad (IV)$$

en présence d'un solvant organique et en présence d'une base.

5. Utilisation des triazolylméthyl-oxirannes substitués de formule (I) en tant que produits intermédiaires de la synthèse de substances possédant une activité fongicide et régulatrice de la croissance des végétaux.